# EUROPEAN PATENT APPLICATION

(11) **EP 4 843 488 A1**
(43) Date of publication of application: **25.11.2026**
(21) Application number: 25177214.1
(22) Date of filing: 19.05.2025
(51) Int. Cl.: A61B 5/0215

(54) **AN ADAPTER FOR AN INVASIVE BLOOD MEASUREMENT SYSTEM**

(71) Applicant: Everest Acquisition Entity, LLC, Wilmington, DE 19805 (US)
(72) Inventor: LI, Jingming, 5656 AG Eindhoven (NL); JIN, Rui, 5656 AG Eindhoven (NL); CHAN, Kwan Ho, 5656 AG Eindhoven (NL); WANG, Aihua, 5656 AG Eindhoven (NL); CHEN, Chaobin, 5656 AG Eindhoven (NL); FU, Hongping, 5656 AG Eindhoven (NL)
(74) Representative: Weilnau, Carsten

(57) **Abstract**

A mechanism for facilitating the identification of a number of transducer output interfaces that are connected to an adapter having three or more adapter input interfaces. Each adapter input interface comprises a first adapter input pin connected to a first adapter output pin by a first conductive element. Each adapter input interface also comprises a second adapter input pin connected to a second adapter output pin by a second conductive element. For each adapter input interface, the combined resistance of the first and second conductive element is unique to that adapter input interface.

## Description

### FIELD OF THE INVENTION

The present invention relates to the field of medical systems.

### BACKGROUND OF THE INVENTION

Invasive blood pressure monitoring systems are widely used in medical settings to provide continuous, real-time measurements of a subject's blood pressure. These systems typically involve inserting a catheter into a blood vessel and connecting it to a transducer that converts the pressure signal into an electrical output. The electrical output is then processed and displayed by a measurement device, allowing healthcare professionals to monitor the patient's cardiovascular status.

Traditional invasive blood pressure monitoring setups often require separate connections between each transducer and the measurement device. This can lead to a complex array of cables and connectors, which may be challenging to manage in busy clinical environments. Additionally, the need for multiple individual connections increases the risk of errors during setup and can make it difficult to quickly identify which transducer is associated with each measurement channel.

Another challenge in current systems is the limited flexibility in accommodating different numbers of transducers. Many measurement devices are designed to work with a fixed number of inputs, making it difficult to adapt the system to varying clinical needs or to upgrade existing setups without replacing the entire measurement device.

It has been appreciated that an adapter for invasive blood pressure monitoring systems is needed that overcomes one or more of these problems.

### SUMMARY OF THE INVENTION

The invention is defined by the claims.

In accordance with a proposed approach, there is provided an adapter for connecting three or more transducer output interfaces of an invasive blood measurement system to a measurement device, wherein the adapter comprises: three or more adapter input interfaces, wherein each adapter input interface comprises: a first adapter input pin for connection to a first transducer output pin of a respective transducer output interface; and a second adapter input pin for connection to a second transducer output pin of a respective transducer output interface; and an adapter output interface for connection to the measurement device, wherein the adapter output interface comprises: a first adapter output pin for connection to a processing unit of the measurement device and for connection to a high voltage level in the measurement device; and a second output pin for connection to a ground pin of the measurement device; and for each adapter input interface: a first conductive element connecting the first adapter input pin of said adapter input interface to the first adapter output pin of the adapter output interface; and a second conductive element connecting the second adapter input pin of said adapter input interface to the second output pin of the adapter output interface, wherein, for each adapter input interface, the summed resistance of the first conductive element and the second conductive element for said adapter input interface is different to the summed resistance of the first conductive element and the second conductive element for any other adapter input interface.

This adapter enables connection of multiple transducer output interfaces to a single measurement device, simplifying the setup of invasive blood measurement systems. The unique resistance characteristics for each adapter input interface allow the measurement device to distinguish between different connected transducers. This can allow for selective processing or use of sensing signals provided from the adapter input interface, e.g., to avoid processing of noise or non-existent signals.

In some examples, the adapter further comprises a resistive element connected between the first adapter output pin and the second output pin. The addition of a resistive element between the first and second output pins facilitates identification (by the measurement device) of whether or not the adapter is connected to the measurement device. In particular, the resistive element will cause a voltage at a first measurement input pin (when connected to the first adapter output pin) to change, thereby allowing for the measurement device to distinguish between a connection and nonconnection to the adapter.

In some examples, the resistance of the resistive element is greater than the summed resistance of the first conductive element and the second conductive element for said adapter input interface.

In some examples, for each adapter input interface: the respective first conductive element comprises a resistor connecting the first adapter input pin of said adapter input interface to the first adapter output pin of the adapter interface; and the resistance of the resistor for said adapter input interface is different to the resistance of the resistor for any other adapter input interface. Using distinct resistors for each adapter input interface allows for precise identification of the connection of individual transducer output interfaces. This arrangement facilitates accurate identification as to which adapter input interfaces are connected to a respective transducer output interface.

In some examples, for each adapter input interface, a ratio of the summed resistance of the first conductive element and the second conductive element for said adapter input interface and the summed resistance of the first conductive element and the second conductive element for each other adapter input interface is outside of the range 0.8 to 1.2. Ensuring a significant difference in resistance ratios between adapter input interfaces improves the reliability of detection of a connection between an adapter input interface and a transducer output interface. This design choice minimizes the risk of misidentification due to small variations in component tolerances or environmental factors.

In some examples, each adapter input interface comprises one or more data adapter input pins for connection to a respective one or more data transducer output pins of the respective transducer output interface; and the adapter output interface comprises, for each data adapter input pin, a respective data adapter output pin. The inclusion of data pins allows for the transmission of a (e.g., differential) sensing signal from any connected transducer output interface to the measurement device. This feature facilitates the monitoring and analysis of invasive blood pressure data.

In some examples, for each adapter input interface, the summed resistance of the first conductive element and the second conductive element for said adapter input interface is no less than 10kΩ. Using relatively high resistance values helps to minimize power consumption in the adapter.

In accordance with another proposed approach, there is provided a measurement device for connecting to the previously described adapter. The measurement device comprises: a measurement input interface comprising: a first measurement input pin for connection to the first adapter output pin of the adapter output interface; and a ground pin for connection to the second output pin of the adapter output interface, wherein the ground pin is connected to a ground voltage; a pull-up resistor connecting the first measurement input pin to the high voltage level; and the processing unit communicatively connected to the first measurement input pin, wherein the processing unit is configured to monitor a voltage at the first measurement input pin to predict a number of transducer output interfaces connected to the three or more adapter input interfaces of the adapter.

This measurement device design enables automatic detection and counting of connected transducers (i.e., detection and identification of which transducer input interfaces are connected to respective transducer output interfaces). The ability to predict the number of connected transducers without manual input streamlines the setup process and reduces the risk of configuration errors in clinical settings.

In some examples, the processing unit is configured to process the voltage at the first measurement input pin using a look-up table to map the voltage at the measurement input pin to a predicted number of transducer output interfaces. Using a look-up table for voltage-to-transducer mapping provides a fast and efficient method for determining the number and/or identifying connected transducers. This approach allows for quick system initialization and can accommodate various adapter configurations without requiring complex calculations.

In some examples, the processing unit is configured to process the voltage at the first measurement input pin to predict, for each adapter input interface of the adapter, whether or not said adapter input interface is connected to a transducer output interface. The ability to detect the connection status of individual adapter input interfaces allows for real-time monitoring of transducer connections, enabling prompt identification of disconnected or malfunctioning sensors, e.g., during medical procedures.

In some examples, the measurement input interface further comprises, for each data adapter output pin of the adapter output interface, a respective data measurement input pin for connection to the data adapter output pin. The inclusion of data measurement input pins allows for the reception of the sensing signal(s) from the transducers.

There is also provided a transducer measurement system for the invasive blood measurement system comprising the adapter and the measurement device.

In some examples, , for each adapter input interface: the respective first conductive element comprises a resistor connecting the first adapter input pin of said adapter input interface to the first adapter output pin of the adapter interface; and the resistance of the resistor for said adapter input interface is different to the resistance of the resistor for any other adapter input interface; and the resistance of the resistive element of the adapter is more than 5 times greater than the resistance of the pull-up resistor of the measurement device.

In some examples, the transducer measurement system further comprises one or more transducer output interfaces, wherein each transducer output interface comprises: a transducer input arrangement configured to connect to a transducer for monitoring an invasive blood pressure; and a transducer output arrangement comprising: the first transducer output pin for connection to the first adapter input pin of a respective adapter input interface; and the second transducer output pin for connection to the second adapter input pin of the respective adapter input interface, wherein the first transducer output pin is directly electrically connected to the second transducer output pin.

In some examples, each adapter input interface comprises one or more data adapter input pins for connection to a respective one or more data transducer output pins of the respective transducer output interface; and the adapter output interface comprises, for each data adapter input pin, a respective data adapter output pin; and for each transducer output interface, the transducer output arrangement comprises a respective data transducer output pin for each data adapter input pin of the respective adapter input interface.

There is also provided an invasive blood measurement system comprising: the transducer measurement system and for each transducer output interface, a respective transducer for monitoring an invasive blood pressure.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 schematically illustrates an invasive blood measurement system;
Fig. 2 illustrates a portion of an existing invasive blood measurement system; and
Fig. 3 illustrates a portion of an invasive blood measurement system comprising a proposed adapter and measurement device.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for facilitating the identification of a number of transducer output interfaces that are connected to an adapter having three or more adapter input interfaces. Each adapter input interface comprises a first adapter input pin connected to a first adapter output pin by a first conductive element. Each adapter input interface also comprises a second adapter input pin connected to a second adapter output pin by a second conductive element. For each adapter input interface, the combined resistance of the first and second conductive element is unique to that adapter input interface.

The present disclosure recognizes that, in use, when a transducer output interface is connected to an adapter input interface, the transducer output interface will short the first and second adapter input pins together. Thus, a resistance between the first adapter output pin and the second adapter output pin will change responsive to which adapter input interfaces are connected to a transducer output interface. This change in resistance can be identified and mapped to a corresponding identification of how many and which adapter input interfaces are connected to respective transducer output interfaces.

This detection mechanism enables the measurement device to automatically configure itself for the correct number and arrangement of connected transducers. It eliminates the need for manual configuration or separate detection circuits for each possible transducer connection, simplifying the overall system design and improving reliability.

The ability to map resistance changes to specific connection configurations relies on appropriate design and selection of the resistances in the conductive elements. The combined resistances are different to one another, to ensure that each possible combination of connected adapter input interfaces results in a unique, distinguishable total resistance between the first adapter output pin and the second adapter output pin. This allows the measurement device to unambiguously determine the connection state of the system.

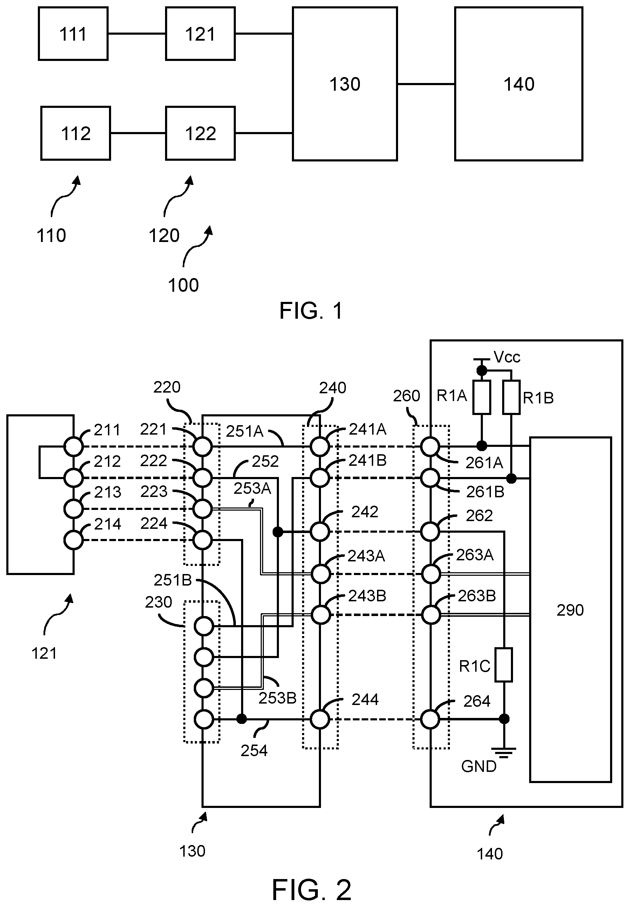

Fig. 1 schematically illustrates components of an existing invasive blood measurement system 100 for the purposes of improved contextual understanding.

The blood measurement system 100 comprises a plurality 110 of transducers 111, 112; a plurality 120 of transducer output interfaces 121, 122; an adapter 130; and a measurement device 140.

Each transducer 111, 112 of the plurality 110 of transducers is configured to convert a blood pressure signal into a sensing signal. Thus, in use, a portion of the transducer 111, 112 (e.g., a catheter) is positioned within a blood vessel (e.g., an artery, such as the radial or femoral artery) and generates a sensing signal responsive to a blood pressure within the blood vessel in which it is positioned. Examples of transducers are widely known and available in the art.

The plurality of transducer output interfaces 121, 122 comprises a respective transducer output interface 121, 122 for each transducer 111, 112. The transducer output interface comprises a transducer input interface for connection to the transducer and a transducer output interface for providing a connection to the adapter 130.

The transducer output interface may be integrated with its respective transducer or be a separate component to said transducer.

In some examples, particularly useful where the transducer output interface is a separate component, the transducer output interface functions as an adapter cable, to adapt different transducers with different connector forms to a unified or predefined connector (for the adapter).

The adapter 130 is designed to connect each transducer output interface to the measurement device. In particular, the adapter 130 may be configured to reduce or consolidate a number of pins or connections of the transducer output interfaces 121, 122 for connection to the measurement device (e.g., consolidate all ground pins of the transducer output interfaces to a single ground pin for connection to the measurement device).

In one existing example, the adapter 130 is configured to connect to only 2 transducer output interfaces, to thereby support 2 channel invasive blood measurements.

The measurement device 140 receives the sensing signals produced by the transducers via the adapter 130. In particular, the measurement device 140 may convert the (analogue) sensing signal from each transducer to digital data, which may be further processed (e.g., for storage, display, analysis and so on).

It is recognized that it would be useful if the measurement device is able to detect the presence of the adapter 130 and/or identify how many transducers are connected to the adapter (e.g., via respective transducer output interfaces).

Fig. 2 provides a more detailed view of a portion of the existing invasive blood measurement system 100 which includes a mechanism for detecting the presence of the adapter and determining how many transducers are connected to the adapter.

Each transducer output interface 121 (of which only one is illustrated) comprises a first transducer output pin 211, a second transducer output pin 212, a set 213 of one or more data transducer output pins (e.g., comprising a pair of pins) and a ground transducer output pin 214.

The first transducer pin 211 and the second transducer output pin 212 are directly electrically connected to one another (i.e., shorted together).

The set of one or more data transducer output pins 213 carries the sensing signal from the associated transducer (not illustrated in Fig. 2). In particular, it is common for a transducer to require a minimum of two dedicated physical connections serving as positive and negative sensing data inputs. These connections are separate from the sensor detection circuits. This means that each transducer (and therefore any set of pins for carrying the sensing signal of said transducer) would benefit from its own pair of data transmission lines for connection.

The ground transducer output pin 214 is for connection to a ground, e.g., an earth or ground reference voltage.

The adapter 130 is configured to connect each transducer output interface 121 to the measurement device 140. In particular, the adapter 130 comprises two adapter input interfaces 220, 230. Each adapter input interface is for connection to a corresponding transducer output interface. Each adapter input interface comprises a first adapter input pin 221, a second adapter input pin 222, a set of one or more data adapter input pins 223, and a ground adapter input pin 224.

The first adapter input pin 221 is for connection to the first transducer output pin 211 of the corresponding transducer output interface. The second adapter input pin 222 is for connection to the second transducer output pin 212 of the corresponding transducer output interface. The set of one or more data adapter input pins 223 is for connection to the set of one or more data transducer output pins 213 of the corresponding transducer output interface.

The adapter 130 also comprises an adapter output interface 240 for connection to the measurement device 140.

The adapter output interface 240 comprises two first adapter output pins 241A, 241B; a second output pin 242; two sets of one or more data adapter output pins 243A, 243B; and a ground adapter output pin 244. Each output pin is designed for connection to a corresponding measurement input pin of the measurement device 140.

The adapter 130 comprises two first connectors 251A, 251B, each connecting a respective first adapter output pin 241 to the first adapter input pin 221 of a respective adapter input interface 220, 230. The adapter 130 comprises a second connector 252 connecting the second adapter output pin 242 to the second adapter input pin 221 of each adapter input interface. The adapter also comprises, for each set of one or more data adapter output pins 243A, 243B a respective data connector 253A, 253B, each connecting a respective set of one or more data adapter output pin 243A, 243B to the set of one or more data adapter input pins 223 of a respective adapter input interface 220, 230. The adapter also comprises a ground connector 254 connecting the ground adapter input pin 224 to the ground adapter output pin 244 of each adapter input interface.

The measurement device 140 comprises a measurement input interface 260 comprising: two first measurement input pins 261A, 261B for connection to the two first adapter output pins 241A, 241B of the adapter output interface 240; a second measurement input pin 262 for connection to the second output pin 242 of the adapter output interface; two sets of one or more data measurement input pins 263A, 263B for connection to the sets of one or more data adapters output pins 243A, 243B of the adapter output interface; and a ground measurement input pin 264 for connection to the ground adapter output pin 244 of the adapter input interface.

The second measurement input pin 262 connects to a ground GND via a resistor R1C. The ground measurement input pin 264 connects to a ground GND (e.g., an earth or reference voltage), preferably directly but optionally via a resistor (not illustrated in Fig. 2). Each other pin 261A, 261B, 262A, 262B of the measurement input interface 260 connects to a processing unit 290 of the measurement device 140.

The processing unit 290 is adapted to process signals received at the two first measurement input pins and the second measurement input to detect how many transducer output interfaces 121 are connected to the adapter 130. More specifically, the processing unit 290 is adapted to identify, for each adapter input interface 220, 230, whether or not a corresponding transducer output interface 121 is connected thereto.

The processing unit 290 is also adapted to receive any signals received at the sets of data measurement input pins, i.e., receive any sensing signals. In particular, the processing unit 290 may be configured to only process the sensing signal(s) for which it has been detected a corresponding transducer output interface is connected to the adapter.

An existing procedure for identifying for each adapter input interface 220, 230, whether or not a corresponding transducer output interface 121 is connected thereto is hereafter described for contextual understanding. This procedure makes use of the identified structure and layout of the transducer output interface(s) 121 and the adapter 130 previously described and illustrated (by Fig. 2).

In the measurement device, a first resistor R1A connects between a high voltage Vcc (e.g., 3V, 3.3V or 5V) and the first measurement input pin 261A. A second resistor R1B connects between the high voltage Vcc and the second measurement input pin. The resistance of the first and second resistor(s) may be fairly large, e.g., > 50 kΩ, e.g., 100 kΩ.

It will be appreciated that each adapter input interface 220, 230 is associated with a respective pair of first measurement input pin and data measurement input pin.

In this configuration (assuming that the adapter 130 and the measurement device 140 are correctly connected together), for each adapter input interface 220, 230, if there is no corresponding transducer output interface connected thereto, then the voltage at the associated first measurement input pin will be pulled to the high voltage Vcc.

Otherwise, the voltage at the associated first measurement input pin will be pulled to ground GND. In particular, when a transducer output interface is connected to an adapter input interface, a current path between the associated first measurement input pin is formed (via the shorting of the first transducer pin 211 and the second transducer output pin 212).

Accordingly, the processing unit is able to monitor the voltage at each first measurement input pin and determine whether or not the associated transducer output interface is connected to the adapter (e.g., a high voltage indicates no connection, and a low/ground voltage indicates a connection).

Thus, from the foregoing, it will be clear that the existing system requires the adapter output interface of the adapter to comprise a respective first adapter output pin for each transducer input interface. Increasing the number of adapter interfaces for the adapter will thereby inherently increase the number of pins on the adapter output interface, which may be disadvantageous (e.g., as the output interface may no longer align with an existing/standard pin structure or arrangement).

The present disclosure provides an improved adapter that overcomes at least some of these problems.

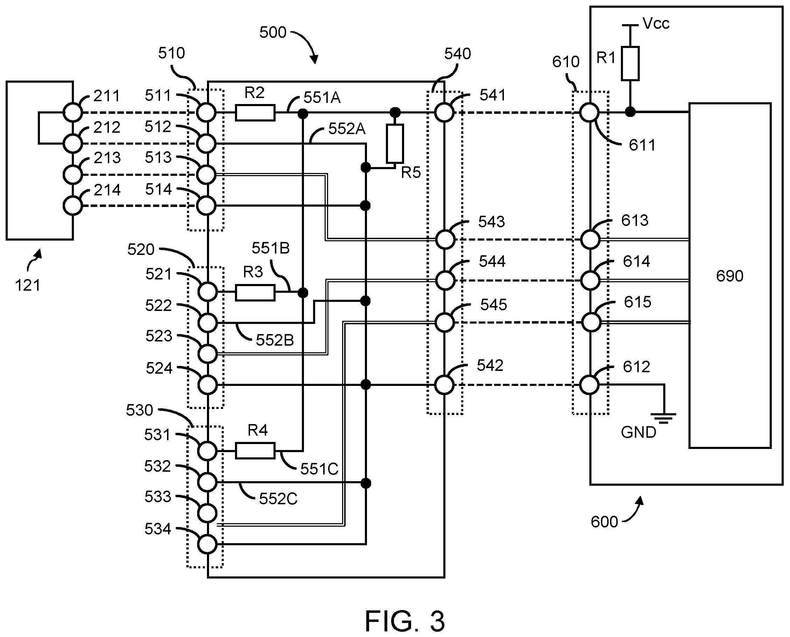

Fig. 3 illustrates a portion of an invasive blood measurement system comprising a proposed adapter 500. The adapter 500 is designed for connecting three or more transducer output interfaces 121, 122, 123 to a measurement device 600. The measurement device 600 is, itself, a proposed embodiment to compliment the proposed adapter 500.

The adapter 500 comprises three or more adapter input interfaces 510, 520, 530. Each adapter input interface is configured for connection to a respective transducer output interface. In the proposed adapter design, each adapter input interface effectively represents a different channel for connecting transducer output interfaces to the measurement device.

In the illustrated example, the adapter 500 comprises only three adapter input interfaces, but may comprise additional adapter input interfaces in other examples. More specifically, the adapter comprises (at least) a first adapter input interface 510; a second adapter input interface 520 and a third adapter input interface 530.

Each adapter input interface comprises a first adapter input pin 511, 521, 531 for connection to a first transducer output pin 211 of a respective transducer output interface. Each adapter input interface also comprises a second adapter input pin 512, 522, 532 for connection to a second transducer output pin of a respective transducer output interface.

The adapter 500 also comprises an adapter output interface 540 for connection to the measurement device 600.

The adapter output interface 540 comprises a first adapter output pin 541 for connection to a processing unit 690 of the measurement device 600 and for connection to a high voltage level Vcc in the measurement device (e.g., via a measurement input interface of the measurement device). The adapter output interface 540 also comprises a second output pin 542 for connection to a ground pin 612 of the measurement device 600.

The adapter further comprises, for each adapter input interface 510, 520, 530, a first conductive element 551A, 551B, 551C connecting the first adapter input pin 511, 521, 531 of said adapter input interface to the first adapter output pin 541 of the adapter output interface 540.

The adapter also comprises, for each adapter input interface 510, 520, 530, a second conductive element 552A, 552B, 552C connecting the second adapter input pin 512, 522, 532 of said adapter input interface 510, 520, 530 to the second output pin 542 of the adapter output interface 540.

Thus, the adapter comprises, for each adapter input interface, a respective pair of a first conductive element and a second conductive element.

It will be appreciated that, when a transducer output interface is connected to the adapter input interface, the first conductive element and the second conductive element for said adapter input interface become serially connected (via the shorting of the first transducer pin 211 and the second transducer output pin 212).

Correspondingly, when no transducer output interface is connected to the adapter input interface, the first conductive element and the second conductive element for said adapter input interface is not (directly) electrically connected together (as there is an open circuit between the first adapter input pin and the second adapter input pin).

The adapter is adapted or configured wherein, for each adapter input interface, the summed resistance of the first conductive element and the second conductive element for said adapter input interface is different to the summed resistance of the first conductive element and the second conductive element for any other adapter input interface.

In other words, the combined resistance of each pair of first and second conductive elements for an adapter input interface is different to the combined resistance of each pair of first and second conductive elements for any other adapter input interface.

In this way, the total resistance between the first adapter output pin 541 of the adapter output interface and the second output pin 542 of the adapter output interface will change dependent upon which (if any) adapter input interfaces are connected to respective transducer output interfaces.

This facilitates identification, by a measurement device 600, of which adapter input interfaces are connected to respective transducer output interfaces.

In particular, the measurement device 600 is configured to comprise a measurement input interface 610 comprising: a first measurement input pin 611 for connection to the first adapter output pin 541 of the adapter output interface 540; and a ground pin 612 for connection to the second output pin 542 of the adapter output interface 540. The ground pin 612 is connected to a ground voltage GND.

The measurement device 600 also comprises a pull-up resistor R1 connecting the first measurement input pin to the high voltage level Vcc.

The voltage at the first measurement input pin (when the measurement device is connected to the adapter) will thereby depend upon which (if any) adapter input interfaces 510, 520, 530 are connected to a respective transducer output interface 121.

Put another way, the unique resistance characteristics associated with each adapter input interface will create distinct voltage levels at the first measurement input pin 611 when different combinations of transducer output interfaces are connected. By analyzing this voltage, the measurement device 600 is able to determine how many and which specific adapter input interfaces are actively connected to transducer output interfaces.

Thus, the measurement device may comprise a processing unit 690 communicatively connected to the first measurement input pin, wherein the processing unit is configured to monitor a voltage at the first measurement input pin to predict at least a number of transducer output interfaces connected to the three or more adapter input interfaces of the adapter, and preferably identify which specific adapter input interfaces are actively connected to transducer output interfaces.

In particular, an effective voltage divider is formed from the pull-up resistance R1 and the resistance between the first adapter output pin 541 of the adapter output interface and the second output pin 542 of the adapter output interface. By monitoring the value of the voltage at the first measurement input pin 611, it is therefore possible to identify which (if any) adapter input interfaces are connected to respective transducer output interfaces. The voltage divider thereby effectively functions as a resistance detector.

Consider a scenario in which the pull-up resistor has a value of A, the combined resistance of a first pair of first and second conductive elements (for the first adapter input interface 510) is B, the combined resistance of a second pair of first and second conductive elements (for the second adapter input interface 520) is C and the combined resistance of a third pair of first and second conductive elements (for the third adapter input interface 530) is D.

In this scenario, the voltage V(611) at the first measurement input pin 611 will be as follows:

**TABLE 1**

| Connections (to any TOIs) | V(611) |
|---|---|
| No AII connected | Vcc |
| 1^{st} AII only | $Vcc \frac{B}{A + B}$ |
| 2^{nd} AII only | $Vcc \frac{C}{A + C}$ |
| 3^{rd} AII only | $Vcc \frac{D}{A + C}$ |
| 1^{st} and 2^{nd} AII only | $Vcc \frac{\frac{BC}{B + C}}{A + \frac{BC}{B + C}}$ |
| 1^{st} and 3^{rd} AII only | $Vcc \frac{\frac{BD}{B + D}}{A + \frac{BD}{B + D}}$ |
| 2^{nd} and 3^{rd} AII only | $Vcc \frac{\frac{CD}{C + D}}{A + \frac{CD}{C + D}}$ |
| 1^{st}, 2^{nd} and 3^{rd} AII | $Vcc \frac{\frac{BCD}{BC + BD + CD}}{A + \frac{BCD}{BC + BD + CD}}$ |

The initialism "AII" means adapter input interface and the initialism "TOI" means transducer output interface.

It will be appreciated, from Table 1, that the voltage at the first measurement input pin 611 will, assuming the values of B, C and D differ, indicate which connection scenario exists, and thereby which adapter input interfaces are connected to transducer output interfaces. In this context, a "connection scenario" represents which adapter input interfaces are connected to a respective transducer output interface.

In other words, this approach enables the measurement device to determine which specific adapter input interfaces are connected to transducer output interfaces, effectively identifying active and inactive channels. An active channel is one where an adapter input interface is connected to a transducer output interface, allowing for the transmission of sensing signals from the transducer to the measurement device. In contrast, an inactive channel may refer to an adapter input interface that is not connected to any transducer output interface, and thus does not transmit or carry any meaningful sensing data.

The processing unit 690 may be configured to map a voltage at the first measurement input pin 611 to a connection scenario (e.g., following equation 1) to thereby determine how many (and which) adapter input interfaces are connected to respective transducer output interfaces.

Put another way, the processing unit 690 may be configured to process the voltage at the first measurement input pin 611 using a look-up table to map the voltage at the measurement input pin to a predicted number of transducer output interfaces. More specifically, the processing unit may be configured to process the voltage at the first measurement input pin 611 using a look-up table to map the voltage to an identification of which (if any) adapter input interfaces are connected to a respective transducer output interface.

In a working example, the look-up table may map each of a plurality of predetermined voltages to a respective identification of which (if any) adapter input interfaces are connected to a respective transducer output interface.

The processing unit may be configured to identify a closest one (in value) of the predetermined voltages to the voltage at the first measurement input pin and identify the corresponding identification of which (if any) adapter input interfaces are connected to a respective transducer output interface.

For instance, the processing unit may compare the actual voltage at the first measurement input pin to a set of predetermined voltages stored in the look-up table. It then selects the closest matching predetermined voltage, accounting for small variations due to component tolerances or environmental factors. Once the closest predetermined voltage is identified, the processing unit retrieves the corresponding information from the look-up table. This information specifies which adapter input interfaces, if any, are connected to transducer output interfaces. By using this method, the processing unit is able to reliably determine the connection status of each adapter input interface without requiring an exact voltage match.

To facilitate improved distinction and discrimination between the different connection scenarios, the values of the combined resistance of the pairs of first and second conductive elements should be sufficiently different, e.g., such that the possible voltage at the first measurement pin 611 will be evenly distributed across the range of possible voltages. This is analogous to setting or defining different quantization levels of an analogue-to-digital converter, to facilitate increased accuracy in identifying the correct connection scenario. By choosing resistance values that result in well-separated voltage levels for different connection scenarios, the measurement device will be able to more accurately or reliably determine which adapter input interfaces are connected to transducer output interfaces.

In some examples, for each adapter input interface, a ratio of the summed resistance of the first conductive element and the second conductive element for said adapter input interface and the summed resistance of the first conductive element and the second conductive element for each other adapter input interface is outside of the range 0.8 to 1.2.

This separation in resistance values translates to distinct voltage levels at the first measurement input pin, improving the reliability of the connection detection mechanism. It reduces the likelihood of misidentification due to small variations in component tolerances, environmental factors, or measurement noise.

The choice of the 0.8 to 1.2 range provides a balance between ensuring clear differentiation and allowing for practical implementation. A wider range (e.g., 0.5 to 2.0) would provide even greater separation but might lead to impractically large differences in resistance values across the adapter input interfaces. Conversely, a narrower range might not provide sufficient distinction between connection scenarios, potentially leading to detection errors, but may be useful in low voltage scenarios.

In some examples, for each pair of first and second conductive elements, the difference between the combined resistance of said pair and each other pair is preferably no less than a quarter of the smallest combined resistance of each pair of first and conductive elements.

Thus, in some examples: |B-C| ≥ 0.25*min(B, C, D); |B-D| ≥ 0.25*min(B, C, D) and |C-D ≥ 0.25*min(B, C, D), where |·| is the absolute magnitude function and min(·) is a minimum function that selects the smallest of the enclosed parameters.

As a working example, the value of the combined resistance of the first pair of first and second conductive elements (for the first adapter input interface 510) may be 12.4 kΩ, the value of the combined resistance of a second pair of first and second conductive elements (for the second adapter input interface 520) may be 16.5 kΩ and the value of the combined resistance of the third pair of first and second conductive elements (for the third adapter input interface 530) may be 28.7 kΩ.

Other approaches for determining suitable values for the combined resistances of the pairs of first and second conductive elements will be apparent. For instance, the resistance values may be determined by continually trying them within a certain range and calculating the corresponding voltages for the first measurement input pin, and then selecting the combination of resistances which has the maximum gap in voltage.

In some examples, at least one of the first and second conductive elements, of each pair of first and second conductive elements, comprises a resistor.

In the illustrated example, for each adapter input interface 510, 520, 530, the respective first conductive element comprises a resistor R2, R3, R4 connecting the first adapter input pin of said adapter input interface to the first adapter output pin of the adapter interface. In such examples, the second conductive elements is absent an (intentional) resistor, beyond the usual wire resistance of a connection.

The resistance of the resistor for each adapter input interface is different to the resistance of the resistor for any other adapter input interface.

However, other variants and configurations for positioning a resistor for each pair of first and second conductive elements will be apparent to the appropriately skilled person.

In some examples, as illustrated, the adapter 500 may further comprise a resistive element R5 (i.e., a resistor) connected between the first adapter output pin 541 and the second output pin 542. This allows for the measurement device to automatically identify whether or not the adapter is connected to the measurement device, i.e., to discriminate between a scenario in which no adapter is connected (when the voltage V(611) at the first measurement input pin 611 will be pulled to the high voltage level) and an adapter is connected, but no transducer output interfaces are connected (when the voltage V(611) at the first measurement input pin 611 will be divided across the pull-up resistor R1 and the resistive element R5).

Thus, the measurement device may be configured to discriminate between a first scenario in which no adapter is connected to the measurement input interface 610 and a second scenario in which the adapter is connected to the measurement input interface 610, but no transducer output interface 121 is connected to the adapter 500, by monitoring the voltage at the first measurement input pin 611.

In particular, the processing unit may be configured to determine that the first scenario is present (i.e., no adapter is connected to the measurement input interface 610) when the voltage at the first measurement input pin 611 is at the high voltage level Vcc and that the second scenario is present (i.e., the adapter 500 is connected to the measurement input interface 610, but no AII is connected to any TOIs) when the voltage at the first measurement input pin 611 is less than the high voltage level Vcc. The ability to discriminate between these two scenarios has not been previously achieved, and allows for improved operation of the measurement device 600 (e.g., to prepare components of the measurement device for connection, via a connected adapter, to an adapter cable).

By way of example, the processing unit 690 may be configured to only begin or trigger one or more preparatory processes (e.g., activating ADCs or allocating processing resource) when it determines, through monitoring the voltage at the first measurement input pin 611, that an adapter is connected to the measurement input interface 610.

In this way, the processing unit 690 may be configured to monitor a voltage at the first measurement input pin 611 to predict whether or not an adapter 500 is connected to the measurement input interface 610. In particular, when the voltage at the first measurement input pin is equal to (e.g., within a predetermined error margin of) the high voltage level Vcc, it may be predicted that the adapter 500 is disconnected from the measurement input interface. Otherwise, it may be assumed that the adapter 500 is connected to the measurement input interface 610.

Moreover (e.g., if/when it is determined that an adapter is connected to the measurement input pin 611), the processing unit may be configured to process the voltage at the first measurement input pin to predict, for each adapter input interface of the adapter, whether or not said adapter input interface is connected to a transducer output interface, e.g., using the relationships detailed in Table 2 below. This follows a similar procedure to that previously disclosed.

Assuming the voltage of the resistive element R5 has a value E, the voltage V(611) at the first measurement input pin 611 will be as follows:

**TABLE 2**

| Connections (to any TOIs) | V(611) |
|---|---|
| No AII connected | $Vcc \frac{E}{A + E}$ |
| 1^{st} AII only | $Vcc \frac{\frac{BE}{B + E}}{A + \frac{BE}{B + E}}$ |
| 2^{nd} AII only | $Vcc \frac{\frac{CE}{C + E}}{A + \frac{CE}{C + E}}$ |
| 3^{rd} AII only | $Vcc \frac{\frac{DE}{D + E}}{A + \frac{DE}{D + E}}$ |
| 1^{st} and 2^{nd} AII only | $Vcc \frac{\frac{BCE}{BC + BE + CE}}{A + \frac{BCE}{BC + BE + CE}}$ |
| 1^{st} and 3^{rd} AII only | $Vcc \frac{\frac{BDE}{BD + BE + DE}}{A + \frac{BDE}{BD + BE + DE}}$ |
| 2^{nd} and 3^{rd} AII only | $Vcc \frac{\frac{CDE}{CD + CE + DE}}{A + \frac{CDE}{CD + CE + DE}}$ |
| 1^{st}, 2^{nd} and 3^{rd} AII | $Vcc \frac{\frac{BCDE}{BCD + BCE + BDE + CDE}}{A + \frac{BCDE}{BCD + BCE + BDE + CDE}}$ |

The resistance of the pull-up resistor R1 is preferably much smaller than the resistance of the resistive element R5 that connects the first adapter output pin 541 and the second output pin 542. In particular, the value of the resistive element R5 may be no less than five times greater (e.g., no less than ten times greater) than the resistance of the pull-up resistor R1. This provides a broader range of possible values for the voltage at the first measurement input pin 611, for ease of separating or distinguishing the voltage levels for different combinations of connected adapter input interfaces from one another.

It will be appreciated that, in some examples, each adapter input interface 510, 520, 530, comprises a set 513, 523, 533 of one or more data adapter input pins for connection to a respective set of one or more data transducer output pins of the respective transducer output interface.

Similarly, the adapter output interface 540 may comprise, for each set 513, 523, 533 of one or more data adapter input pins, a respective set 543, 544, 545 of one or more data adapter output pins. Correspondingly, the measurement input interface may further comprise, for each set of one or more data adapter output pins 543, 544, 545 of the adapter output interface, a respective set 613, 614, 615 of one or more data measurement input pins for connection to the respective set of one or more data adapter output pins.

This provides a mechanism for carrying or transferring a sensing signal from each (connected) transducer output interface to the measurement device 600.

It will be appreciated that each set 613, 614, 615 of one or more data measurement input pins is thereby associated with a corresponding adapter input interface. For instance, a first set 613 is associated with a first adapter input interface 510, a second set 614 is associated with a second adapter input interface 520 and a third set 615 is associated with a third adapter input interface 530. Each set will be connected to the respective data adapter input pin(s) of its corresponding adapter input interface when the adapter is connected to the measurement device.

In some examples, the processing unit 690 may be configured to selectively process any (sensing) signals received at each set 613, 614, 615 of one or more data measurement input pins based on the determination of whether the corresponding adapter input interface is connected to a transducer output interface.

In some examples, the processing unit 690 may be configured to selectively process (sensing) signals received at each set of each set 613, 614, 615 of one or more data measurement input pins based on the determination of whether the corresponding adapter input interface 510, 520, 530 is connected to a transducer output interface 121, 122, 123. This selective processing may be implemented as follows:
The processing unit 690 may first analyze the voltage at the first measurement input pin 611 to determine which adapter input interfaces 510, 520, 530 are connected to transducer output interfaces 121, 122, 123, as previously described. Once this determination is made, the processing unit 690 may use this information to control its processing of signals received at each set 613, 614, 615 of one or more data measurement input pins.

For each set 613, 614, 615 of one or more data measurement input pins, the processing unit 690 may check whether the corresponding adapter input interface 510, 520, 530 has been determined to be connected to a transducer output interface 121, 122, 123. If a connection is detected, the processing unit 690 may enable or activate the signal processing for that particular set of data measurement input pins. Conversely, if no connection is detected for a given adapter input interface, the processing unit 690 may disable or not process any signals received at the corresponding set of data measurement input pins.

This helps conserve computational resources by focusing on only active channels, whilst also preventing the processing of noise or irrelevant signals from disconnected channels, potentially improving the overall accuracy and reliability of the blood pressure measurements.

The processing unit 690 may implement this selective processing through a software and/or hardware mechanism. For example, it may use software-controlled switches or multiplexers to route signals from connected channels to its processing algorithms. Alternatively, it may employ conditional statements in its software to skip processing steps for disconnected channels.

In some examples, the processing unit 690 may also provide feedback to the user about which channels are active, based on this determination. This information could be displayed on a user interface, helping operators to verify proper connections and troubleshoot any issues with the setup of the invasive blood measurement system.

In some examples, each adapter input interface 510, 520, 530 also comprises a ground adapter input pin 514, 524, 534. Each ground adapter input pin may be connected to the second adapter output pin 612 (which, in turn, is for connection to a ground GND). This provides a common reference point for the electrical signals within each adapter input interface, which is useful for maintaining signal integrity and reducing noise in the system.

Proposed embodiments of the measuring device comprise a processing unit. The processing unit may comprise one or more processing modules. Suitable examples of processing modules include: general purpose microcontroller units (MCU); field programmable gate arrays (FPGAs); application-specific integrated circuits (ASICs); digital signal processors (DSPs); or other specialized processing modules designed for specific tasks. The choice of specific one or more processing modules for the processing unit may depend on factors such as computational requirements, power consumption constraints, and the need for real-time performance in the context of network communication.

In some instances, the processing unit may comprise one or more analogue-to-digital converters (ADCs). In particular, if present, each set of one or more data measurement input pins may connect to the one or more ADCs, which may convert any sensing signal carried thereover into digital data (e.g., for processing by the processing module).

The ADC(s) may also function to enable the processing unit to identify different voltage levels at the first measurement input pin 611. More specifically, the processing unit 290 of the existing measurement device 140 (Fig. 2) may only be able to discriminate between a high and low voltage level, rather than a more granular voltage level that allows for identifying different connection scenarios.

Thus, the processing unit may comprise an analogue-to-digital converter (ADC) for converting the voltage at the first measurement input pin to digital data processable by the processing module(s) of the processing unit.

The number of ADCs in the processing unit may, for instance, depend upon the capability of each ADC. For instance, the processing unit may comprise a first ADC for connection to the first measurement input pin 611 (e.g., to convert a voltage at the measurement pin to digital data) and a respective second ADC for each set of one or more data measurement input pins 613, 614, 615. Alternatively, a single combined ADC may be employed for both functions/uses.

A wide variety of uses for the sensing signals, received by the processing unit, are known in the art. For instance, the sensing signals may be processed to capture or produce sensing data (e.g., representing a measured blood pressure). This sensing data may be stored, display (e.g., for viewing by a clinician) and/or further processed (e.g., combined with other forms of data to produce relevant medical data). The precise use of the sensing signals is immaterial to the underlying proposed concept.

It will be appreciated that there is also herein proposed a transducer measurement system comprising the proposed adapter and the proposed measurement device.

The transducer measurement system may further comprise one or more transducer output interfaces. Each transducer output interface comprises: a transducer input arrangement configured to connect to a transducer for monitoring an invasive blood pressure; and a transducer output arrangement.

Examples of suitable transducer output arrangements have been previously described. In particular, each transducer output arrangement may comprise the first transducer output pin for connection to the first adapter input pin of a respective adapter input interface; and the second transducer output pin for connection to the second adapter input pin of the respective adapter input interface, wherein the first transducer output pin is directly electrically connected to the second transducer output pin.

Of course, for each transducer output interface, the transducer output arrangement may comprise one or more respective data transducer output pin for connecting to the corresponding one or more data adapter input pin of the respective adapter input interface.

It has been previously described how a transducer may require a minimum of two dedicated physical connections serving as positive and negative sensing data lines (e.g., to carry the sensing signal as a differential signal). Accordingly, each set of data pins (e.g., each set 513, 523, 533 of data adapter input pins, each set 543, 544, 545 of data adapter output pins and each set 613, 614 614 of data measurement input pins) may comprise a corresponding pair of pins to accommodate the positive and negative sensing data lines from the transducer.

The proposed measurement system 600 may also be configured to be (backwards-)compatible with the existing adapter 140 (Fig. 2).

It is noted, as previously explained, that each set of one or more data measurement input pins 613, 614, 615 may, in practice, comprise a pair of data measurement input pins.

When the proposed measurement device 600 is used with the existing adapter 140, one of the two first adapter output pins 241A of the adapter 140 may connect to the first measurement input pin 261A and the other of the first adapter output pins 241B of the adapter 140 may connect to a first one of a first set 613 of data measurement input pins. The second output pin 242 of the adapter 140 may connect to a second one of the first set of data measurement input pins.

When no adapter input interface is connected to any transducer output interface, the value at the first measurement input pin 261A is pulled to the high voltage level Vcc, whilst the voltage level at each of the first set of data measurement input pins is not pulled to the high voltage level Vcc (e.g., is floating or noisy). The processing unit may correspondingly identify the connection scenario correctly (i.e., no adapter input interface is connected).

For the existing adapter 140, when one (or both) of the adapter input interfaces is connected to a transducer output interface, then the voltage at one (or both) of the first set of data measurement input pins will be pulled to the high voltage level Vcc. This voltage level is different to the expected voltage level of a sensing signal, and can be detected by the measurement unit (thereby indicating that the existing adapter 140 is used instead of the herein proposed adapter 500 (Fig. 3)). In this way, the processing unit is able to automatically identify that the existing adapter 140 is in use.

The measurement device 600 is also able to distinguish or identify which adapter input interface is connected to a respective transducer output interface. In particular, if the voltage at the first one of the first set of data measurement input pins is pulled to the high voltage level Vcc, then the first adapter interface 220 is connected to a transducer output interface. Similarly, if the voltage at the second one of the first set of data measurement input pins is pulled to the high voltage level Vcc, then the second adapter interface 230 is connected to a transducer output interface.

The operation of the other elements of the measurement device 600 and adapter 140 may be similar or identical to those previously disclosed.

This backwards compatibility feature allows users to continue using their existing adapters with the new measurement device, providing flexibility and cost-effectiveness in upgrading systems. The voltage detection mechanism enables automatic identification of the adapter type and connection status, simplifying the setup process and reducing user error.

There is also proposed an invasive blood measurement system comprising the transducer measurement system; and for each transducer output interface, a respective transducer for monitoring an invasive blood pressure. Suitable examples of transducers are well known in the art. In general, each transducer may be configured to produce a sensing signal for transmission over a pair of data lines (e.g., which may connect to the measurement device via the set(s) of data pins in each intervening component.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processing unit may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An adapter (500) for connecting three or more transducer output interfaces (121) of an invasive blood measurement system to a measurement device (600), wherein the adapter comprises:
three or more adapter input interfaces (510, 520, 530), wherein each adapter input interface comprises:
a first adapter input pin (511, 521, 531) for connection to a first transducer output pin (211) of a respective transducer output interface; and
a second adapter input pin (512, 522, 532) for connection to a second transducer output pin (212) of a respective transducer output interface; and
an adapter output interface (540) for connection to the measurement device, wherein the adapter output interface comprises:
a first adapter output pin (541) for connection to a processing unit (690) of the measurement device and for connection to a high voltage level (Vcc) in the measurement device; and
a second output pin (542) for connection to a ground pin (612) of the measurement device; and
for each adapter input interface:
a first conductive element (551A, 551B, 551C) connecting the first adapter input pin of said adapter input interface to the first adapter output pin of the adapter output interface; and
a second conductive element (552A, 552B, 553C) connecting the second adapter input pin of said adapter input interface to the second output pin of the adapter output interface,
wherein, for each adapter input interface, the summed resistance of the first conductive element and the second conductive element for said adapter input interface is different to the summed resistance of the first conductive element and the second conductive element for any other adapter input interface.

2. The adapter of claim 1, further comprising a resistive element (R5) connected between the first adapter output pin and the second output pin.

3. The adapter of claim 2, wherein, for each adapter interface, the resistance of the resistive element (R5) is greater than the summed resistance of the first conductive element and the second conductive element for said adapter input interface.

4. The adapter of any one of claims 1 to 3, wherein, for each adapter input interface:
the respective first conductive element comprises a resistor (R2, R3, R4) connecting the first adapter input pin of said adapter input interface to the first adapter output pin of the adapter interface; and
the resistance of the resistor for said adapter input interface is different to the resistance of the resistor for any other adapter input interface.

5. The adapter of any one of claims 1 to 4, wherein, for each adapter input interface, a ratio of the summed resistance of the first conductive element and the second conductive element for said adapter input interface and the summed resistance of the first conductive element and the second conductive element for each other adapter input interface is outside of the range 0.8 to 1.2.

6. The adapter of any one of claims 1 to 5, wherein:
each adapter input interface comprises one or more data adapter input pins (513, 523, 533) for connection to a respective one or more data transducer output pins of the respective transducer output interface; and
the adapter output interface comprises, for each data adapter input pin, a respective data adapter output pin (543, 544, 545).

7. A measurement device (600) for connecting to the adapter (500) of any one of claims 1 to 6, wherein the measurement device comprises:
a measurement input interface (610) comprising:
a first measurement input pin (611) for connection to the first adapter output pin (541) of the adapter output interface (540); and
a ground pin (612) for connection to the second output pin (542) of the adapter output interface, wherein the ground pin is connected to a ground voltage (GND);
a pull-up resistor (R1) connecting the first measurement input pin to the high voltage level (Vcc); and
the processing unit (690) communicatively connected to the first measurement input pin, wherein the processing unit is configured to monitor a voltage at the first measurement input pin to predict a number of transducer output interfaces connected to the three or more adapter input interfaces of the adapter.

8. The measurement device of claim 7, wherein the processing unit is configured to process the voltage at the first measurement input pin using a look-up table to map the voltage at the measurement input pin to a predicted number of transducer output interfaces.

9. The measurement device of any one of claims 7 to 8, wherein the processing unit is configured to process the voltage at the first measurement input pin to predict, for each adapter input interface of the adapter, whether or not said adapter input interface is connected to a transducer output interface.

10. The measurement device of any one of claims 7 to 9 for connecting to the adapter of claim 5, wherein:
the measurement input interface further comprises, for each data adapter output pin (543, 544, 545) of the adapter output interface, a respective data measurement input pin (613, 614, 615) for connection to the data adapter output pin.

11. A transducer measurement system for the invasive blood measurement system comprising the adapter of any one of claims 1 to 6 and the measurement device of any one of claims 7 to 10.

12. The transducer measurement system of claim 11, wherein:
the adapter is the adapter of claim 3; and
the resistance of the resistive element of the adapter is more than 5 times greater than the resistance of the pull-up resistor of the measurement device.

13. The transducer measurement system of claim 11 or 12, further comprising one or more transducer output interfaces, wherein each transducer output interface comprises:
a transducer input arrangement configured to connect to a transducer for monitoring an invasive blood pressure; and
a transducer output arrangement comprising:
the first transducer output pin for connection to the first adapter input pin of a respective adapter input interface; and
the second transducer output pin for connection to the second adapter input pin of the respective adapter input interface,
wherein the first transducer output pin is directly electrically connected to the second transducer output pin.

14. The transducer measurement system of claim 13, wherein:
the adapter is the adapter of claim 5; and
for each transducer output interface, the transducer output arrangement comprises a respective data transducer output pin for each data adapter input pin of the respective adapter input interface.

15. An invasive blood measurement system comprising:
the transducer measurement system of any one of claims 13 or 14; and
for each transducer output interface, a respective transducer for monitoring an invasive blood pressure.
